# EUROPEAN PATENT SPECIFICATION

(11) **EP 3 198 278 B1**
(45) Date of publication and mention of the grant of the patent: **10.03.2021**
(21) Application number: 15844912.4
(22) Date of filing: 24.09.2015
(51) Int. Cl.: G01N 33/574, G01N 33/53, C12Q 1/68, G01N 33/50

(54) **CIRCULATING TUMOR CELL DIAGNOSTICS FOR IDENTIFICATION OF RESISTANCE TO ANDROGEN RECEPTOR TARGETED THERAPIES**
DIAGNOSTIKA MIT ZIRKULIERENDEN TUMORZELLEN ZUR IDENTIFIZIERUNG DER RESISTENZ GEGEN AUF DEN ANDROGENREZEPTOR GERICHTETE THERAPIEN
DIAGNOSTIC BASÉ SUR LES CELLULES TUMORALES CIRCULANTES POUR L'IDENTIFICATION D'UNE RÉSISTANCE AUX THÉRAPIES CIBLANT LES RÉCEPTEURS DES ANDROGÈNES

(30) Priority: 25.09.2014 US 201462055491 P
(43) Date of publication of application: 02.08.2017
(73) Proprietor: Epic Sciences, Inc., San Diego, CA 92121 (US)
(72) Inventor: DITTAMORE, Ryan, San Diego, CA 92121 (US)
(74) Representative: Jones Day
(86) International application number: PCT/US2015/051899
(87) International publication number: WO 2016/049291

(56) References cited:
- WO-A1-2014/008155
- WO-A1-2015/112999
- US-A1- 2012 276 555
- US-A1- 2013 130 241
- US-A1- 2013 171 642
- US-A1- 2013 171 642
- R.M. BAMBURY ET AL: "237PCHARACTERISTICS OF DE NOVO RESISTANCE TO ANDROGEN TARGETING THERAPEUTICS (AR TX) THROUGH CIRCULATING TUMOR CELLS (CTCS) ANALYSIS IN METASTATIC CASTRATION RESISTANT PROSTATE CANCER (MCRPC) PATIENTS", ANNALS OF ONCOLOGY., vol. 25, no. suppl_4, 1 September 2014 (2014-09-01), pages iv79-iv79, XP55450387, NL ISSN: 0923-7534, DOI: 10.1093/annonc/mdu326.70
- H.I. SCHER ET AL: "238PCHARACTERIZATION OF CIRCULATING TUMOR CELLS (CTCS) OF METASTATIC CASTRATION RESISTANT PROSTATE CANCER (MCRPC) PATIENTS IN FIRST, SECOND & THIRD LINE SYSTEMIC THERAPIES", ANNALS OF ONCOLOGY., vol. 25, no. suppl_4, 1 September 2014 (2014-09-01), pages iv79-iv80, XP055450393, NL ISSN: 0923-7534, DOI: 10.1093/annonc/mdu326.71
- Ryan Dittamore ET AL: "Molecular characterization of circulating tumor cells (CTC) and CTC subpopulations in progressive metastatic castration resistant prostate cancer (mCRPC).", Journal of Clinical Oncology, Vol 32, No 4_suppl, 1 February 2014 (2014-02-01), XP055381016, Retrieved from the Internet: URL:http://ascopubs.org/doi/abs/10.1200/jc o.2014.32.4_suppl.132 [retrieved on 2017-06-13]
- STANBROUGH ET AL.: 'Prostatic intraepithelial neoplasia in mice expressing an androgen receptor transgene in prostate epithelium.' PROC NATL ACAD SCI USA. vol. 98, no. 19, 2001, pages 10823 - 8, XP002558208
- MOSTAGHEL ET AL.: 'Molecular Pathways: Targeting Resistance in the Androgen Receptor for Therapeutic Benefit.' CLIN CANCER RES. vol. 20, no. 4, February 2014, pages 791 - 8, XP055220529
- MIYAMOTO ET AL.: 'Androgen receptor signaling in circulating tumor cells as a marker of hormonally responsive prostate cancer.' CANCER DISCOV. vol. 2, no. 11, 2012, pages 995 - 1003, XP055211802
- GUO ET AL.: 'A New Trick of an Old Molecule: Androgen Receptor Splice Variants Taking the Stage?!' INT J BIOL SCI. vol. 7, no. 6, 2011, pages 815 - 822, XP055422451
- KODIHA ET AL.: 'Computer-based fluorescence quantification: a novel approach to study nucleolar biology.' BMC CELL BIOL. vol. 12, no. 25, 2011, page 25, XP021100156

## Description

This application claims the benefit of priority of U.S. provisional application Serial No. 62/055,491, filed September 25, 2014.

The invention relates generally to the field of cancer diagnostics and, more specifically to methods for for prospectively identifying *de novo* resistance to AR targeted therapies in a mCRPC patient.

### BACKGROUND

Prostate cancer (PC) remains the most common non-cutaneous cancer in the US. In 2014 alone, the projected incidence of prostate cancer is 233,000 cases with deaths occurring in 29,480 men, making metastatic prostate cancer therapy truly an unmet medical need. Siegel et al., 2014. CA Cancer J Clin. 2014;64(1):9-29. Epidemiological studies from Europe show comparable data with an estimated incidence of 416700 new cases in 2012, representing 22.8% of cancer diagnoses in men. In total, 92200 PC-specific deaths are expected, making it one of the three cancers men are most likely to die from, with a mortality rate of 9.5%

With the advent of exponential growth of novel agents tested and approved for the treatment of patients with metastatic castration-resistant prostate cancer (mCRPC) in the last 5 years alone, issues regarding the optimal sequencing or combination of these agents have arisen. Several guidelines exist that help direct clinicians as to the best sequencing approach and most would evaluate presence or lack of symptoms, performance status, as well as burden of disease to help determine the best sequencing for these agents. Mohler et al., 2014, J Natl Compr Canc Netw. 2013;11(12):1471-1479; Cookson et al., 2013, J Urol. 2013;190(2):429-438. Currently, approved treatments consist of the taxane class of cytotoxic drugs and androgen-targeted therapies. The challenge for clinicians is to decide the best sequence for administering these therapies to provide the greatest benefit to patients. However, therapy failure remains a significant challenge based on heterogenous responses to therapies across patients and in light of cross-resistance from each agent. Mezynski et al., Ann Oncol. 2012;23(11):2943-2947;. Noonan et al., Ann Oncol. 2013;24(7):1802-1807; Pezaro et al., Eur Urol. 2014, 66(3): 459-465. In addition, patients may lose the therapeutic window to gain substantial benefit from each drug that has been proven to provide overall survival gains. Hence, better methods of identifying the target populations who have the most potential to benefit from targeted therapies remain an important goal.

Circulating tumor cells (CTCs) represent a significant advance in cancer diagnosis made even more attractive by their non-invasive measurement. Cristofanilli et al., N Engl J Med 351:781-91, (2004) CTCs released from either a primary tumor or its metastatic sites hold important information about the biology of the tumor. Quantifying and characterizing CTCs, as a liquid biopsy, assists clinicians to select the course of therapy and to watch monitor how a patient's cancer evolves. CTCs can therefore be considered not only as surrogate biomarkers for metastatic disease but also as a promising key tool to track tumor changes, treatment response, cancer recurrence or patient outcome non-invasively. Historically, the extremely low levels of CTCs in the bloodstream combined with their unknown phenotype has significantly impeded their detection and limited their clinical utility. A variety of technologies are presently being developed developed for detection, isolation and characterization of CTCs in order to utilize their information. Bambury et al.; 2014, Annals of Oncology, vol. 25, no. suppl_4, relates to the characterization of de novo resistance to androgen-targeting therapies through the analysis of AR expression in CTCs from 40 prostate cancer patients.

A need exists to decelop accurate and non-invasive methods for prospective identification of patients with tumors that harbor *de novo r*esistance to AR targeted therapy so as to reduce morbidity and enable early exploration of alternative therapy approaches. The present invention addresses this need by providing a multivariate biomarker predictor of *de novo* resistance to AR targeted therapies based on a robust CTC detection and characterization platform that enables phenotypic characterization of CTCs. Related advantages are provided as well.

### SUMMARY OF THE INVENTION

The present invention provides methods for prospectively identifying *de novo* resistance to AR targeted therapies in a mCRPC patient.

The disclosure provides a method of predicting *de novo* resistance to androgen receptor (AR) targeted therapy in a tumor of a prostate cancer patient comprising (a) performing a direct analysis comprising immunofluorescent staining and morphological characteristization of nucleated cells in a blood sample obtained from the patient to generate circulating tumor cell (CTC) data, wherein the analysis comprises determining a measurable feature of a panel of traditional and non-traditional CTC biomarkers for *de novo* resistance to androgen receptor (AR) targeted therapy , and (c) evaluating the CTC data to determine the probability of *de novo* resistance to the AR targeted therapy in the tumor of the prostate cancer patient. In some aspects, the immunofluorescent staining comprises staining of nucleated cells comprises pan cytokeratin, cluster of differentiation (CD) 45, diamidino-2-phenylindole (DAPI) and AR. In some embodiments, the biomarkers (1) CTC heterogeneity, (2) frequency of cytokeratin positive (CK+), AR N-terminal positive CTCs with prominent nucleoli morphology, and (3) frequency of AR C-terminal truncated CTCs . The method of claim 3, wherein the CTC heterogeneity further comprises biomarkers selected from the group consisting of traditional CTCs, CTC clusters, CK- CTCs, small CTCs, nucleoli CTCs, CK speckled CTCs and the biomarkers listed in Table 1.

The invention provides a method of predicting *de novo* resistance to androgen receptor (AR) targeted therapy in a tumor of a prostate cancer patient comprising
(a) performing a direct analysis comprising immunofluorescent staining and morphological characteristization of nucleated cells in a blood sample obtained from the patient to generate circulating tumor cell (CTC) data, wherein the analysis comprises determining a measurable feature of a panel of traditional and non-traditional CTC biomarkers for *de novo* resistance to androgen receptor (AR) targeted therapy,
   wherein the biomarkers comprise (1) CTC heterogeneity, (2) frequency of cytokeratin positive (CK+), AR N-terminal positive CTCs with prominent nucleoli morphology, and (3) frequency of AR C-terminal truncated CTCs, and
   wherein the CTC heterogeneity comprises biomarkers selected from the group consisting of traditional CTCs, CTC clusters, CK- CTCs, small CTCs, nucleoli+ CTCs, CK speckled CTCs and
(b) evaluating the CTC data to determine the probability of *de novo* resistance to the AR targeted therapy in the tumor of the prostate cancer patient,
wherein (1) greater CTC heterogeneity, (2) increased frequency of CK+, AR N-terminal positive CTCs with prominent nucleoli morphology, or (3) increased frequency of AR C-terminal truncated CTCs, compared to patients responsive to AR therapy, predicts *de novo* resistance to AR therapy

In some embodiments, the immunofluorescent staining comprises staining of nucleated cells comprising pan cytokeratin, cluster of differentiation (CD) 45, diamidino-2-phenylindole (DAPI) and AR.

In some embodiments, the method comprises an initial step of depositing the nucleated cells as a monolayer onto a slide.

In some embodiments, the prostate cancer is metastatic castration resistant prostate cancer (mCRPC).

In some embodiments, the CTC data is generated by fluorescent scanning microscopy. In some embodiments, the microscopy provides a field of view comprising both CTCs and at least 200 surrounding white blood cells (WBCs). In some embodiments, the CTC data is generated by assessing at least 4 million of the nucleated cells.

In some embodiments, the CTCs comprise distinct immunofluorescent staining from surrounding nucleated cells; or wherein the CTCs comprise distinct morphological characteristics compared to surrounding nucleated cells.

In some embodiments, the morphological characteristics comprise one or more of the group consisting of nucleus size, nucleus shape, presence of holes in nucleus, cell size, cell shape and nuclear to cytoplasmic ratio, nuclear detail, nuclear contour, presence or absence of nucleoli, quality of cytoplasm and quantity of cytoplasm.

In some embodiments, the identification of CTCs further comprises comparing intensity of pan cytokeratin fluorescent staining to surrounding nucleated cells.

In some embodiments, the method further comprises an initial step of obtaining a white blood cell (WBC) count for the blood sample; an initial step of lysing erythrocytes in the blood sample; and/or an initial step of depositing nucleated cells from the blood sample as a monolayer on a glass slide. In some embodiments, the method comprises depositing between about 2 million and about 3 million cells onto the glass slide.

In some embodiments, the generation of the CTC data comprises enumeration of CTCs in the blood sample.

In some embodiments, the measurable features are analyzed using a predictive model. In some embodiments, the model is a multivariate model.

Other features and advantages of the invention will be apparent from the detailed description, and from the claims.

### BRIEF DESCRIPTION OF THE DRAWINGS

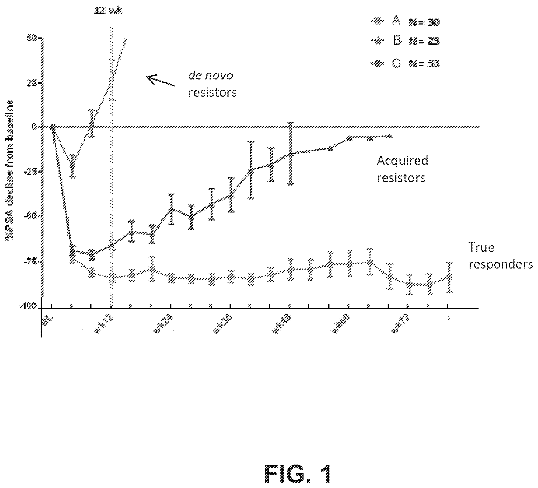

**Figure 1** shows patterns of PSA changes after AR signaling directed therapies (A, True responders, N=30; B, Acquired resistors, N=23; C, *de novo* resistors, N=33).

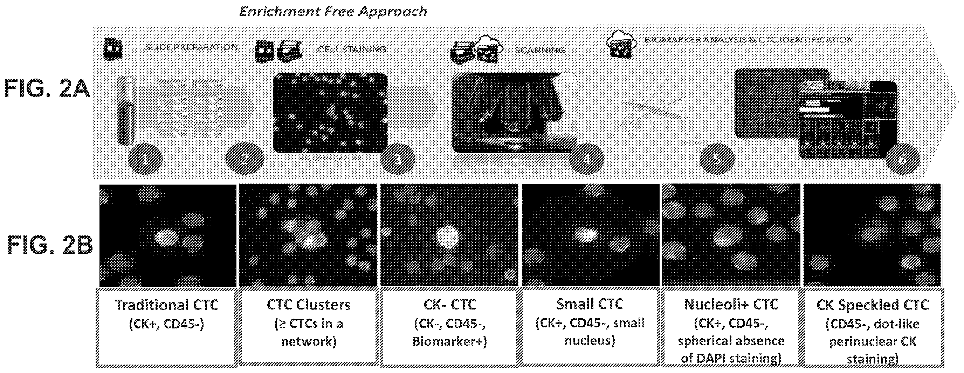

**Figures 2A and 2B** show a schematic of the process and images. Figure 2A shows schematic of Epic's CTC collection and detection process, which flows as follows: (1) Blood lysed, nucleated cells from blood sample placed onto slides; (2) Slides stored in -80C biorepository; (3) Slides stained with CK, CD45, DAPI and AR; (4) Slides scanned; (5) Multi-parametric digital pathology algorithms run, and (6) Software and human reader confirmation of CTCs & quantitation of biomarker expression. Figure 2B shows images of traditional (cytokeratin positive (red), CD45 negative (green), contains a DAPI nucleus (blue), morphologically distinct from surrounding white blood cells) and non-traditional CTCs.

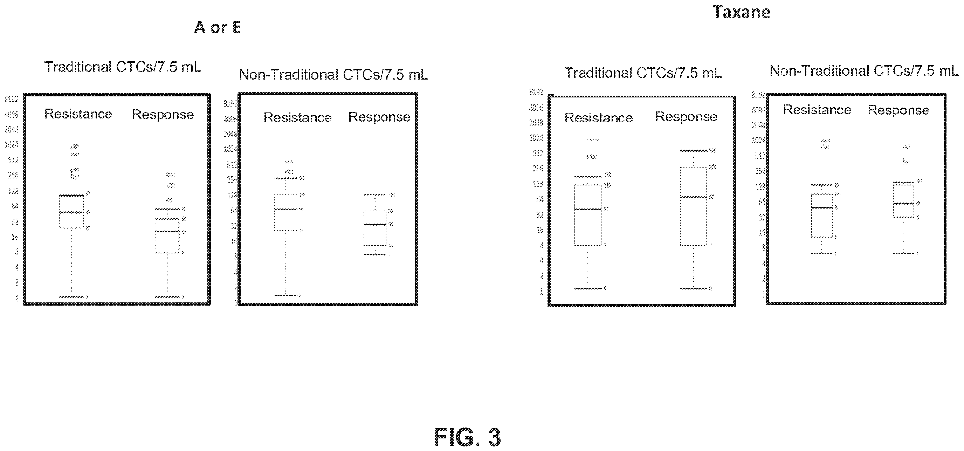

**Figure 3****. CTC Characteristics by AR Therapy Response/Resistance.** Figure 3 shows boxplots representing the number of traditional CTCs, and non-traditional CTCs per 7.5 mL vs. response to A or E (left) or Taxane (right). The box perimeter defines the upper and lower quartiles, the median value is the marker within the box. The whiskers represent 1.5 IQR of the observed values.

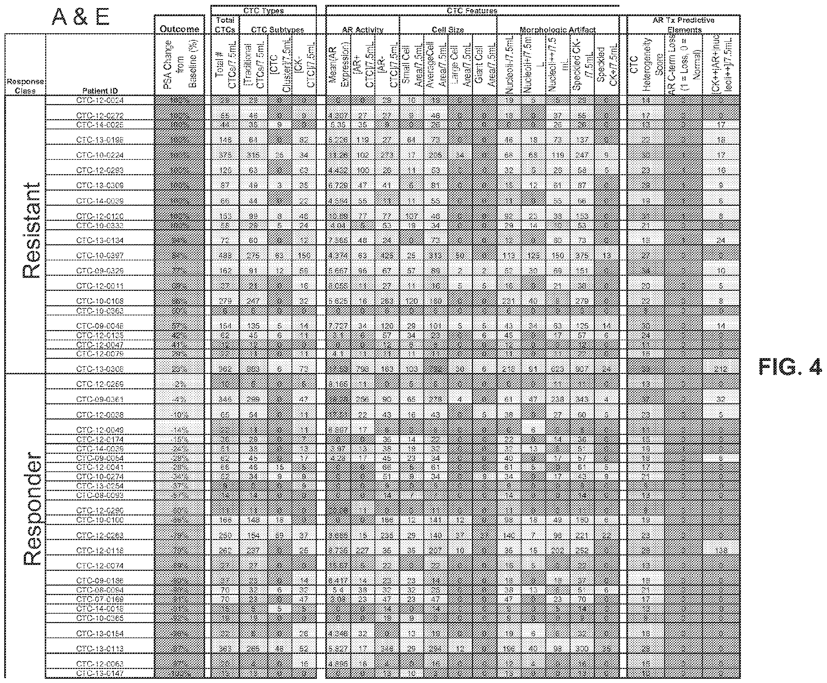

**Figure 4** shows a heat map listing the patients screened prior to 1st ,2nd or 3rd line A or E grouped by response vs. the relative abundance of specific CTC characteristics. CTC features are not mutually exclusive and may not represent unique events. Values displayed are reported in CTC/mL for each phenotype (except for Average AR+ /CTC) and are color coded to reflect relative abundance (high=red/darker gray with positive numbers, mid=yellow/light gray, low=green/darker gray with negative numbers) across all patients.

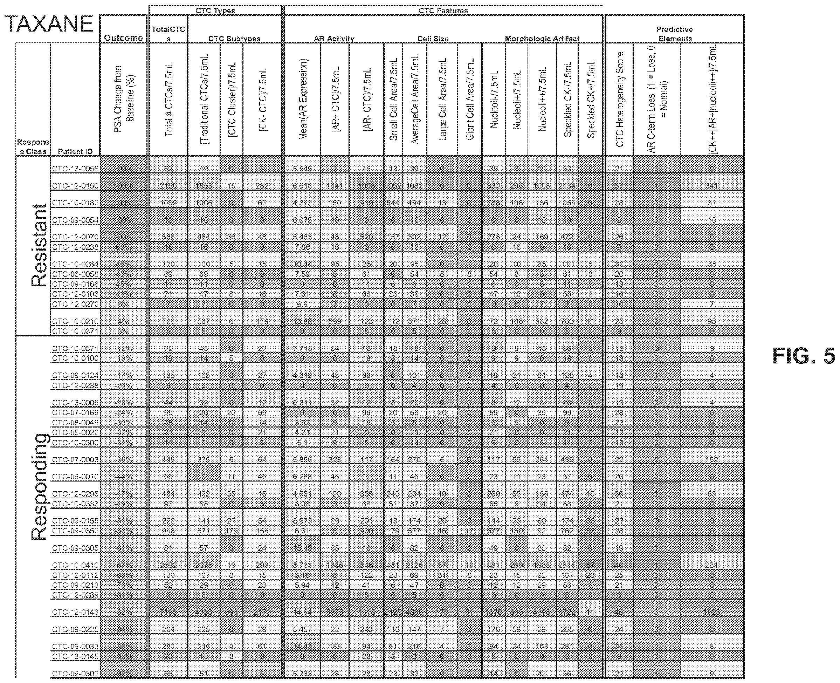

**Figure 5** shows a heat map listing the patients screened prior to 1st ,2nd or 3rd line taxane grouped by response vs. the relative abundance of specific CTC characteristics. CTC features are not mutually exclusive and may not represent unique events. Values displayed are reported in CTC/mL for each phenotype (except for Average AR+ /CTC) and are color coded to reflect relative abundance (high=red/darker gray with positive numbers, mid=yellow/light gray, low=green/darker gray with negative numbers) across all patients.

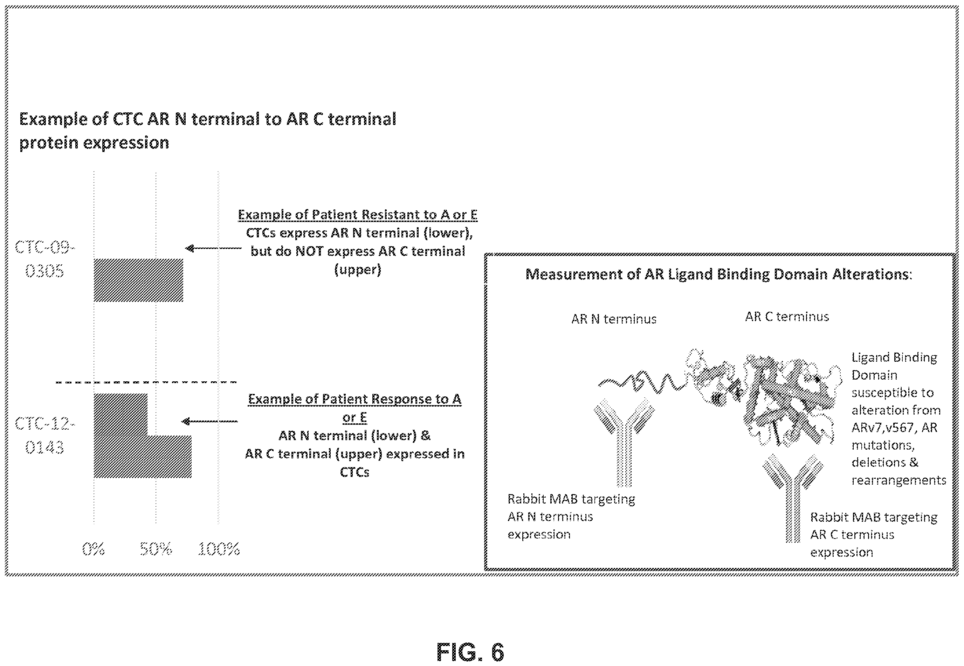

**Figure 6****. Measurement of AR Ligand Binding Domain Alterations.** Figure 6 depicts a bar chart comparing AR expression values between two representative (1 AR Therapy resistant top, 1 AR Therapy responder bottom) patients (both with >40 AR+ CTC/7.5mL) receiving AR therapy. CTCs were examined for AR Ligand Binding Domain (LBD) alterations utilizing a 2 target assay directed at AR N terminal protein expression and AR C terminal protein expression. Loss of AR C-terminal variant expression has been associated with AR Therapy resistance.

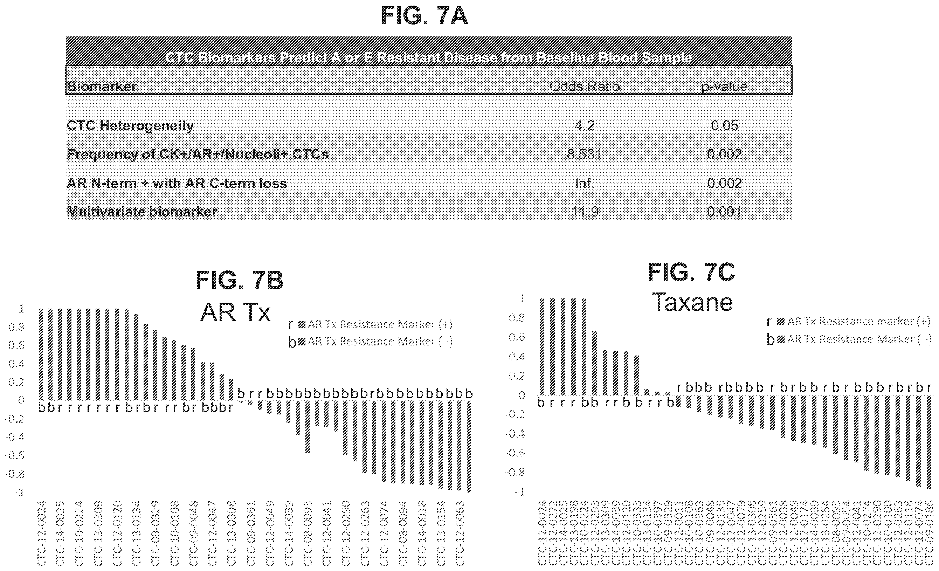

**Figures 7A-7C****. Prediction of AR Therapy *De Novo* Resistance.** Figure 7A shows a table comparing univariate and multivariate models for the prediction of AR Therapy response using molecular and morphological variables. Figures 7B and 7C show waterfall plots depicting the relationship between AR Therapy resistance predication and % Max decrease PSA for patients treated with AR Therapy (Figure 7B) and Taxane (Figure 7C).

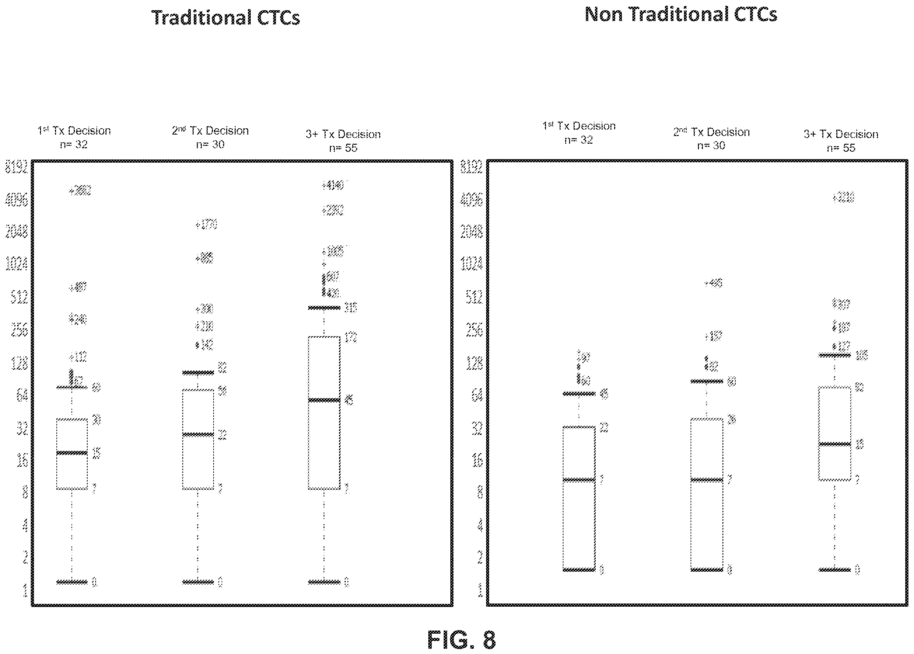

**Figure 8****. CTC Frequency Across 1st, 2nd, or 3rd Line Treatment Decisions.** Figure 8 shows box plots that represent the distribution of CTC/7.5mL values within patients prior to 1st, 2nd and 3rd line treatment with AR Therapy or Taxane therapy. The box perimeter defines the upper and lower quartiles, the median value is the marker within the box. The whiskers represent 1.5 IQR of the observed values..

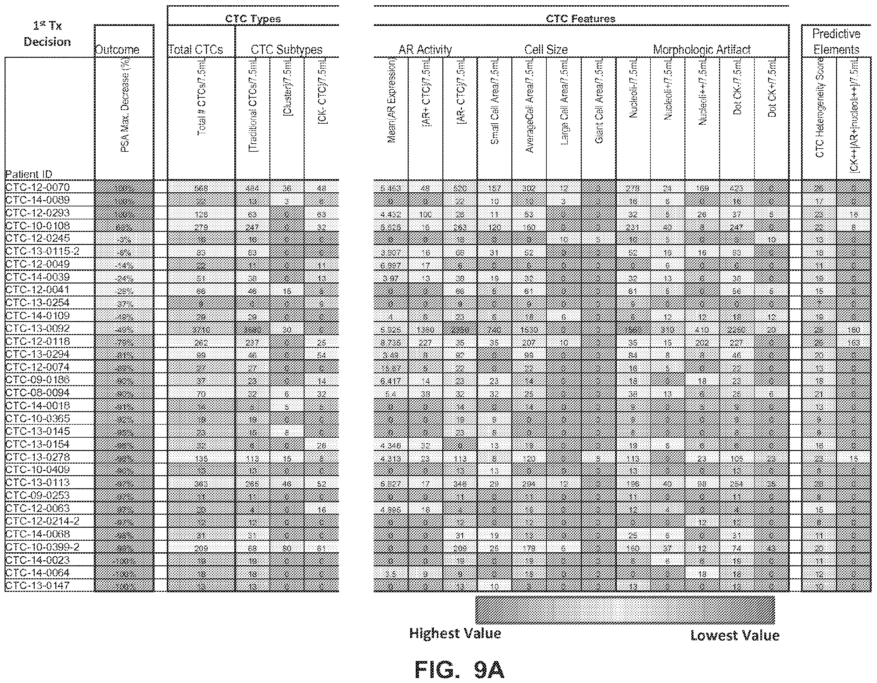

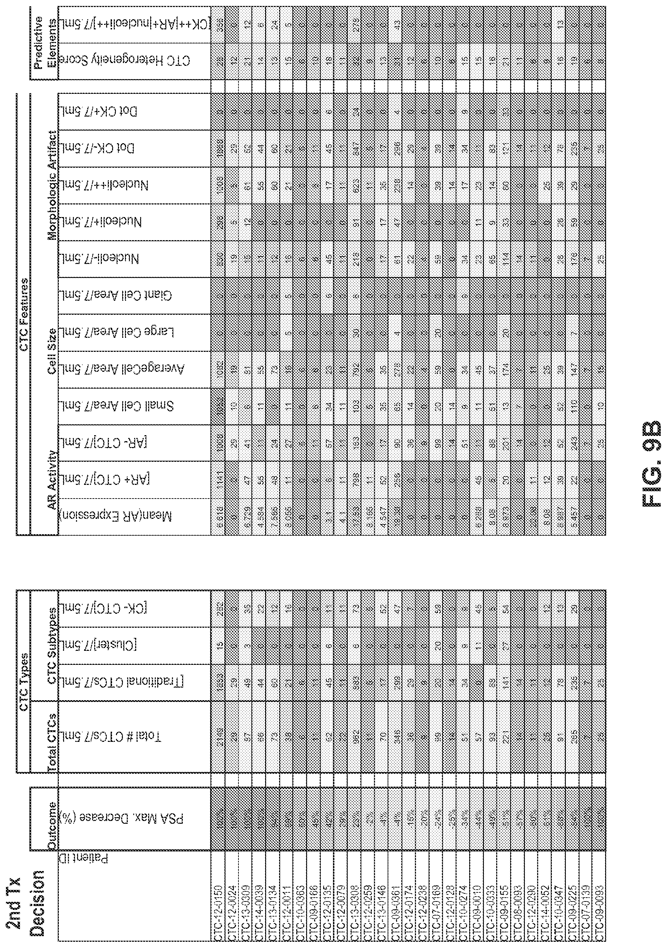

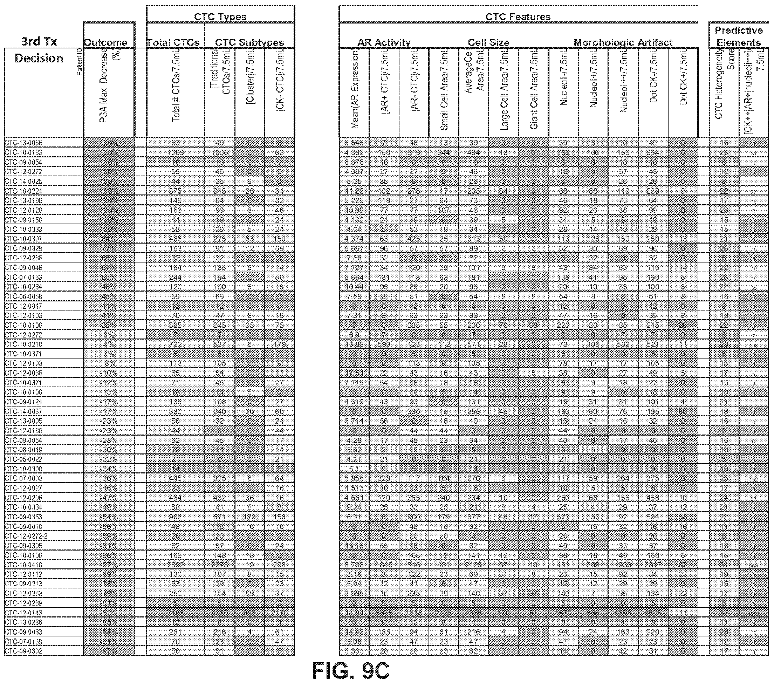

**Figures 9A-9C** show heat maps listing the patients screened prior to 1st (Figure 9A), 2nd (Figure 9B) or 3rd (Figure 9C) line therapy vs. the relative abundance of CTC with specific characteristics (rows). CTC characteristics are not mutually exclusive and may not represent unique events. Values displayed are reported in CTC/7.5 mL for each phenotype (except for Mean AR Expression) and are color coded to reflect relative abundance (high=red/darker gray with positive numbers, mid=yellow/light gray, low=green/darker gray with negative numbers) across all patients.

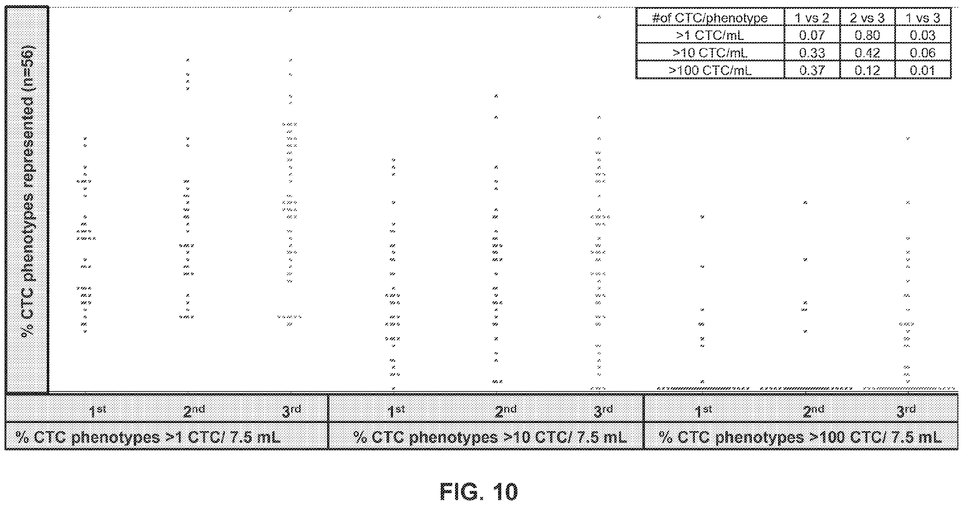

**Figure 10****. Heterogeneity of CTC Phenotypes.** Figure 10 shows a dot plot describing the % of the most common CTC phenotypes represented in patients with greater than 1,10 or 100 CTC/ 7.5 mL at 1st 2nd and 3rd line treatment decisions. The table in the upper right hand corner describes the probability that the observed heterogeneity in CTC subpopulations are not different between groups.

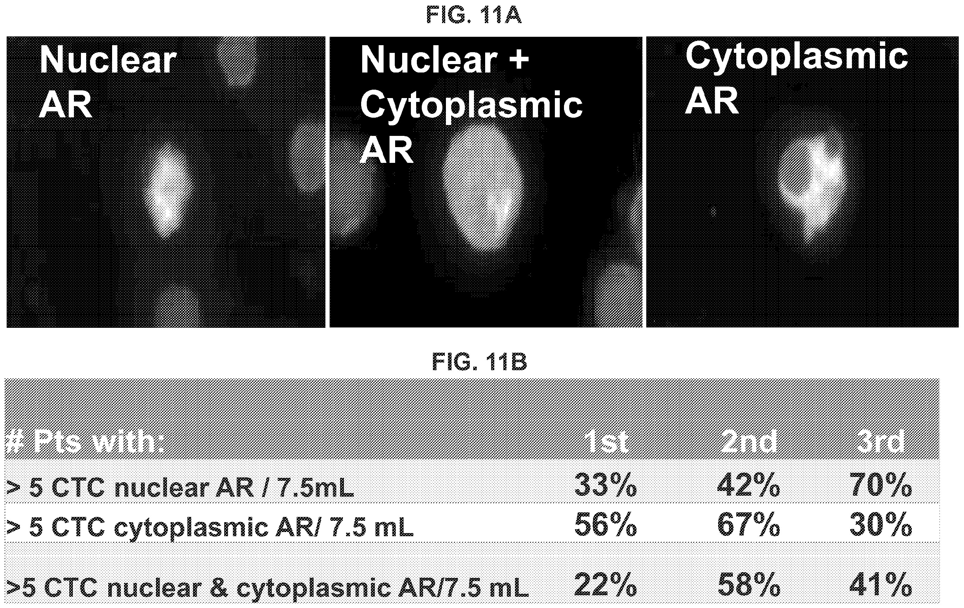

**Figures 11A and 11B****. Heterogeneity of AR Localization.** Figure 11A shows immunofluorescent images of CTCs showing nuclear AR staining, nuclear and cytplasmic AR staining, and cytoplasmic AR staining. Figure 11B is a table describing the distribution of patients with >5 AR positive CTC with AR localized to the nucleus, cytoplasm or both at 1st ,2nd , 3rd line Therapy.

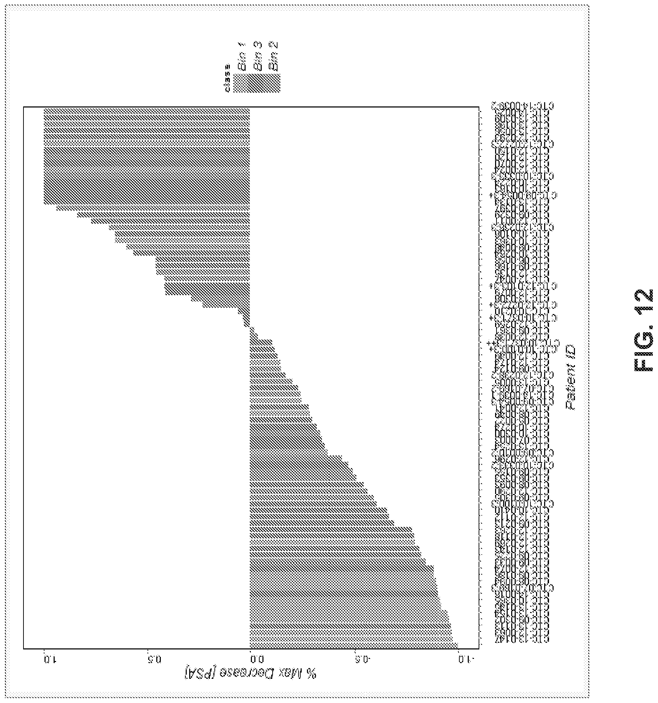

**Figure 12** shows a waterfall plot describing the heterogeneity of response to 1st (salmon/bin 1) 2nd (green/bin 3) or 3rd (blue/bin 2) line therapy as measured by the % maximum decrease in PSA expression.

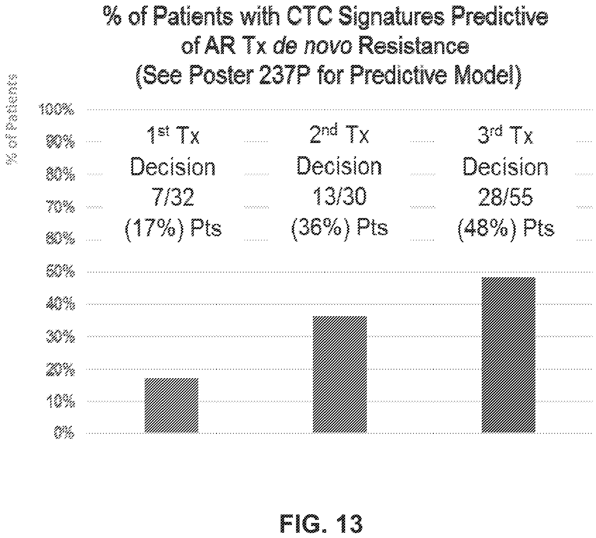

**Figure 13****. CTCs Signatures of AR Therapy Resistance Increase with Successive Therapies.** Figure 13 shows a bar graph identifying the percentage of patients which are predicted to fail AR Therapy (A or E) at 1st, 2nd, and 3rd line treatment decisions. The predictive model utilizes characterization of CTCs as described herein.

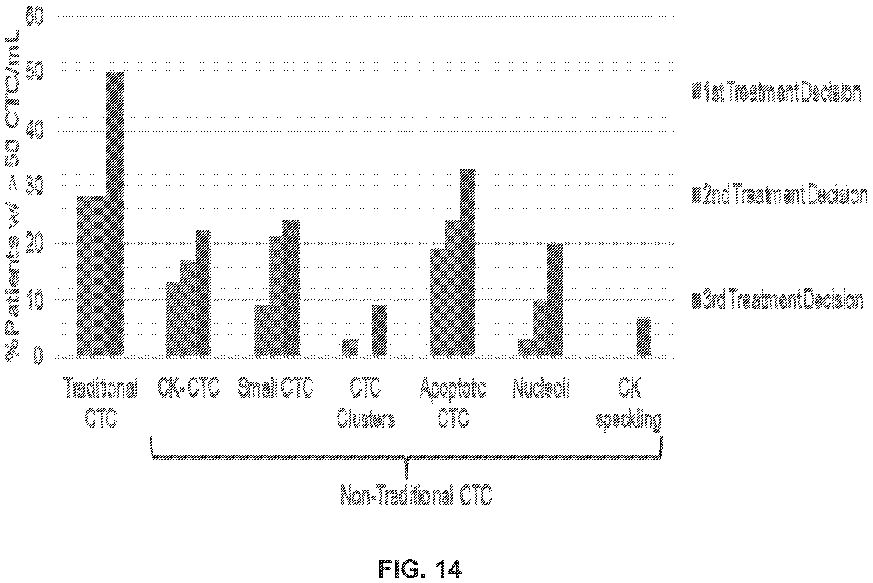

**Figure 14****. Frequency of CTC Phenotypes Increase with Successive Therapies.** Figure 14 shows a bar graph that identifies the percentage of patient facing 1st, 2nd and 3rd line Therapy with >50 CTC events and the particular CTC features associated (each group of bar graphs, from left to right, 1st, 2nd and 3rd treatment decisions).

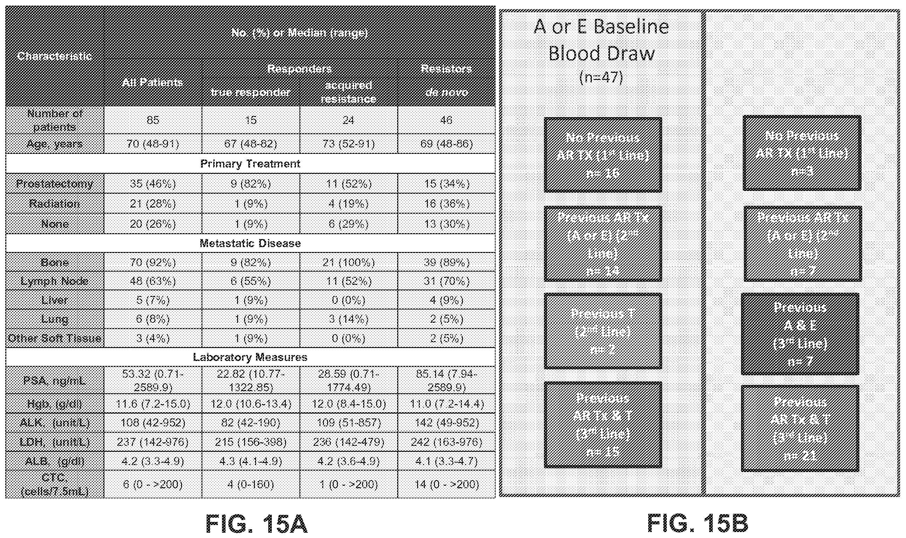

**Figures 15A and 15B.** Figure 15A shows the Study Population for Example 1 and Figure 15B shows previous patient therapies for the population.

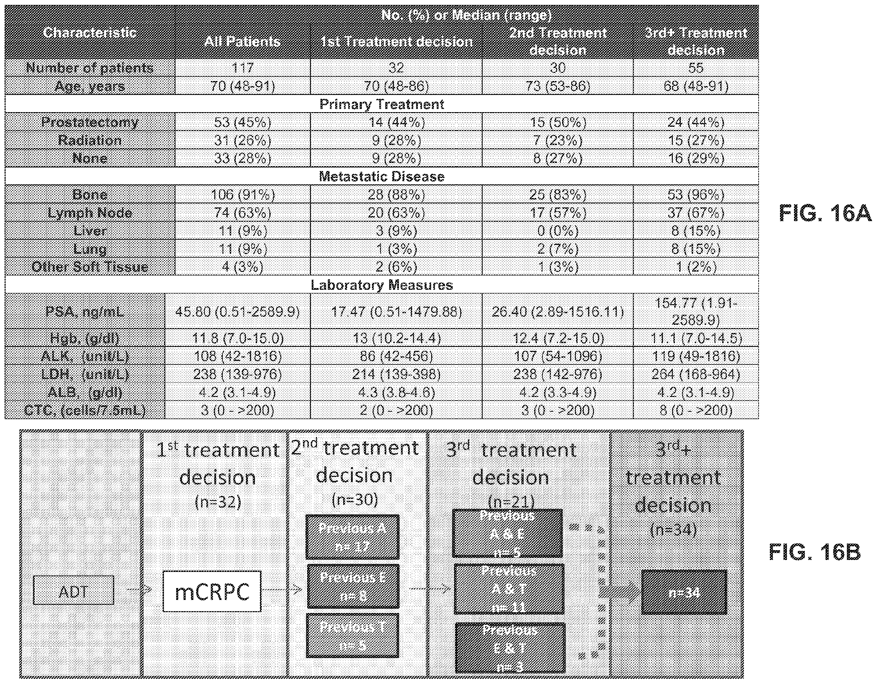

**Figures 16A and 16B.** Figure 16A shows the Study Population for Example 2 and Figure 16B shows clinical treatment decision points in mCRPC.

### DETAILED DESCRIPTION

The present disclosure is based, in part, on the identification of a panel of biomarkers that can prospectively predict *de novo* resistance to AR targeted therapies in a patient afflicted with mCRPC. As disclosed herein, patients not responding to AR targeted therapies demonstrate greater heterogeneity of CTC subpopulations compared to patients who responded to AR targeted therapies. As further disclosed herein, single CTC characterization to measure heterogeneity of CTC subpopulations can be utilized to predict resistance to AR targeted therapies. It is also disclosed herein that epithelial expression, Androgen N-terminal staining and morphologic assessment of CTCs can be utilized to predict response to AR targeted therapies. Additionally, as disclosed herein, the detection of AR C-terminal loss can be utilized to predict resistance to AR targeted therapies.

The methods disclosed herein provide predictive indicators for *de novo* resistance to AR targeted therapy. Identification of predictive biomarkers enables early and ongoing opportunities for therapeutic intervention and adjustments throughout a the clinical progression of mCRPC. Discriminatory multivariate modeling of biomarkers was performed to identify a panel of biomarkers that enables the disclosed methods for identifying a prostate cancer patient with tumors that harbor *de novo* resistance to AR targeted therapy. Accordingly, the present disclosure encompasses a multivariate model to identify a prostate cancer patient with tumors that harbor *de novo* resistance to AR targeted therapy . This disclosure has therefore identified predictive biomarkers of for identifying a prostate cancer patient with tumors that harbor *de novo r*esistance to AR targeted therapy.

In one embodiment, the disclosure provides a method of predicting *de novo* resistance to androgen receptor (AR) targeted therapy in a tumor of a prostate cancer patient comprising (a) performing a direct analysis comprising immunofluorescent staining and morphological characteristization of nucleated cells in a blood sample obtained from the patient to generate circulating tumor cell (CTC) data, wherein the analysis comprises determining a measurable feature of a panel of traditional and non-traditional CTC biomarkers for *de novo* resistance to androgen receptor (AR) targeted therapy , and (c) evaluating the CTC data to determine the probability of *de novo* resistance to the AR targeted therapy in the tumor of the prostate cancer patient. In particular embodiments, the prostate cancer is metastatic castration resistant prostate cancer (mCRPC). In some embodiments, the immunofluorescent staining of nucleated cells comprises pan cytokeratin, cluster of differentiation (CD) 45, diamidino-2-phenylindole (DAPI) and AR.

In some embodiments, the biomarkers used in the methods disclosed herein comprise (1) CTC heterogeneity, (2) frequency of cytokeratin positive (CK+), AR N-terminal positive CTCs with prominent nucleoli morphology, and (3) frequency of AR C-terminal truncated CTCs. In further aspects, CTC heterogeneity comprises CTC biomarkers selected from the group consisting of traditional CTCs, CTC clusters, CK- CTCs, small CTCs, nucleoli ⁺ CTCs and CK speckled CTCs and the additional biomarker set forth in Table 1.

The rapid evolution of drug therapies in prostate cancer has vastly improved upon the use of docetaxel since its pivotal US Food and Drug Administration (FDA) approval in 2004 and has brought about a new era where progress has been made beyond the use of androgen deprivation therapy (ADT) with the addition of novel hormonal agents, immunotherapy, second-line chemotherapy as well as radiopharmaceuticals (see Table 1). The choice of sequencing currently relies on patient profiles, whether symptoms of metastatic disease exist or not.7,8 Men who are asymptomatic or minimally symptomatic may benefit from early use of Sipuleucel-T, while treatment using docetaxel is usually reserved for patients with pain. Radium is used predominantly for patients with bony metastases especially in those who are not candidates for aggressive chemotherapy and abiraterone acetate can be given for effects on pain palliation. Agents such as cabazitaxel, abiraterone acetate, enzalutamide or radium can all be offered after progression on docetaxel. While survival outcomes are undeniably improved with the use of these therapies, disease will ultimately progress on each regimen.

Androgens in the form of testosterone or the more potent dihydrotestosterone (DHT) have been well-defined drivers of progression of prostate cancer and differentiation of the prostate gland. As such, the backbone of treatment for advanced prostate cancers was established decades ago when castration in the form of surgical orchiectomy achieved significant prostate tumor regression.9 Since then, substitution to chemical castration has been employed mostly due to patient preference. 10 ADT has therefore become the standard systemic treatment for locally advanced or metastatic prostate cancer. 11 While ADT is almost always effective in most patients, disease progression to castration resistance inevitably occurs. 12 It is now increasingly recognized that the androgen receptor (AR) remains overexpressed despite seemingly castrate levels of testosterone, since alternative receptors may activate the AR or other target genes may help perpetuate the castrate-resistant phenotype,13,14 hence the term "castration-resistance" has become widely adopted in the literature. The enhanced understanding of the role of these androgens in stimulating the growth of prostate cancer has led to the development and approval of both abiraterone and enzalutamide.

Several factors are associated with increased risk for prostate cancer. Genetics, increasing age, and environmental and geographical factors play a major role. But, dietary factors such as high consumption of fats - fatty acids, alpha linolenic acid found in red meat, etc., deficiency of trace element like selenium and low levels of vitamin D and E have also been implicated in increased risk of development of prostate cancer in some individuals.

It must be noted that, as used in this specification and the appended claims, the singular forms "a", "an" and "the" include plural referents unless the content clearly dictates otherwise. Thus, for example, reference to "a biomarker" includes a mixture of two or more biomarkers, and the like.

The term "about," particularly in reference to a given quantity, is meant to encompass deviations of plus or minus five percent.

As used in this application, including the appended claims, the singular forms "a," "an," and "the" include plural references, unless the content clearly dictates otherwise, and are used interchangeably with "at least one" and "one or more."

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

A "biomarker" is any molecule, property, characteristic or aspect that can be measured and correlated with the probability for prostate cancer, in particular, mCRPC. The term further encompasses any property, characteristic, feature or aspect of a CTC that can be measured and correlated in connection with predicting response to a prostate cancer therapy, for example, AR targeted therapy. For a CTC biomarker, such a measurable feature can include, for example, the presence, absence, or concentration of the biomarker, or a subtype thereof, in the biological sample, an altered immunofuorescent and/or morphological phenotype, such as, for example, nuclear detail, nuclear contour, presence or absence of nucleoli, quality of cytoplasm, quantity of cytoplasm, intensity of immunofluorescent staining patterns in comparison to the appropriate control subjects, and/or the presence or degree of heterogeneity of phenotypes observed for a CTC biomarker. In addition to CTC biomarkers, biomarkers can further include risk indicia including, for example age, family history, race and diet.

As used herein, the term "panel" refers to a composition, such as an array or a collection, comprising one or more biomarkers. The term can also refer to a profile or index of expression patterns of one or more biomarkers described herein. The number of biomarkers useful for a biomarker panel is based on the sensitivity and specificity value for the particular combination of biomarker values.

The term "patient," as used herein preferably refers to a human, but also encompasses other mammals. It is noted that, as used herein, the terms "organism," "individual," "subject," or "patient" are used as synonyms and interchangeably.

As used herein, the term "circulating tumor cell" or "CTC" is meant to encompass any rare cell that is present in a biological sample and that is related to prostate cancer. CTCs, which can be present as single cells or in clusters of CTCs, are often epithelial cells shed from solid tumors found in very low concentrations in the circulation of patients.

As used herein, a "traditional CTC" refers to a single CTC that is cytokeratin positive, CD45 negative, contains a DAPI nucleus, and is morphologically distinct from surrounding white blood cells.

As used herein, a "non-traditional CTC" refers to a CTC that differs from a traditional CTC in at least one characteristic. Non-traditional CTCs include the five CTC subtypes shown in Figure 2, Panel B, including CTC clusters, CK negative CTCs that are positive at least one additional biomarker that allows classification as a CTC , small CTCs, nucleoli⁺ CTCs and CK speckled CTCs. As used herein, the term "CTC cluster" means two or more CTCs with touching cell membranes.

As used herein, the term "CTC heterogeneity" refers to the number and/or frequency of observed CTC subtypes in a sample. CTC subtypes include the non-traditional CTCs described herein as well as any CTCs characterized by morphological and/or immunofluorescence phenotypes that are not associated with traditional CTCs. As disclosed herein, heterogeneity is greater in patients who do not respond to AR targeted therapies compared to patients who respond to AR targeted therapies. Accordingly, a score can be assigned that corresponds to the number of CTC subtypes in a sample . For example, a sample that encompasses every observable CTC subtype will be assigned the maximum heterogeneity score, which can predict that a patient will not respond to AR targeted therapy. Conversely, a lack of heterogeneity corresponds to a low heterogeneity score, which can predict that a patient is likely to respond to AR targeted therapy. A patient's heterogeneity Score can be calculated as the number of biomarkers per 7.5mL of blood sample (Heterogeneity Score (per Patient) = # biomarkers where [biomarker]/7.5mL > 0). CTC heterogeneity is a biomarker useful for practicing the methods of the invention that encompasses multiple measurable features that collectively also serve as biomarkers. Table 1 sets forth a list of hetereogeneity biomarkers useful for practicing the methods of the invention.

| **TABLE 1. HETEROGENEITY BIOMARKERS** |
|---|
| **[CK+ Cluster]/7.5mL** |
| **[CK- Cluster]/7.5mL** |
| **[Cluster]/7.5mL** |
| **[AR+ CTC]/7.5mL** |
| **[AR- CTC]/7.5mL** |
| **[CK+ CTC]/7.5mL** |
| **[CK- CTC]/7.5mL** |
| **Small Cell Area/7.5mL** |
| **AverageCell Area/7.5mL** |
| **Large Cell Area/7.5mL** |
| **Giant Cell Area/7.5mL** |
| **Small Nuclear Area/7.5mL** |
| **Average Nuclear Area/7.5mL** |
| **Large Nuclear Area/7.5mL** |
| **Giant Nuclear Area/7.5mL** |
| **Nucleoli-/7.5mL** |
| **Nucleoli+/7.5mL** |
| **Nucleoli++/7.5mL** |
| **Dot CK-/7.5mL** |
| **Dot CK+/7.5mL** |
| **[CK++\|AR+\|nucleoli++]/7.5mL** |
| **[CK++\|AR-\|nucleoli-]/7.5mL** |
| **[CK++\|AR+\| nucleoli-]/7.5mL** |
| **[CK++\| AR-\|nucleoli++]/7.5mL** |
| **[CK++\|AR+\|nucleoli+]/7.5mL** |
| **[CK++\|AR-\|nucleoli+]/7.5mL** |
| **[CK+\|AR-\|nucleoli++]/7.5mL** |
| **[CK+\|AR-\|nucleoli-]/7.5mL** |
| **[CK++\|AR++\|nucleoli++]/7.5mL** |
| **[CK+\|AR+\|nucleoli++]/7.5mL** |
| **[CK-\|AR-\|nucleoli++]/7.5mL** |
| **[CK-\|AR-\|nucleoli-]/7.5mL** |
| **[CK-\|AR+\|nucleoli+]/7.5mL** |
| **[CK-\|AR+\|nucleoli-]/7.5mL** |
| **[CK+\|AR+\|nucleoli+]/7.5mL** |
| **[CK-\|AR+\|nucleoli++]/7.5mL** |
| **[CK+\|AR+\|nucleoli-]/7.5mL** |
| **\|CK-\|AR-\|nucleoli+]/7.5mL** |
| **[CK-\|AR++\|nucleoli-]/7.5mL** |
| **[CK-\|AR++\|nucleoli++]/7.5mL** |
| **[CK-\|AR++\|nucleoli+]/7.5mL** |
| **[CK+\|AR++\|nucleoli++]/7.5mL** |
| **[CK+**\|**AR++\|nucleoli+]/7.5mL** |
| **[CK++\|AR++\|nucleoli+]/7.5mL** |
| **[CK++\|AR++\|nucleoli-]/7.5mL** |

In some embodiments, the frequency of AR C-terminal truncated CTCs is a biomarker useful for practicing the methods of the invention. As disclosed herein, decrease in nuclear C-terminal AR staining as compared to N- terminal AR nuclear staining can predict that a patient will not respond to AR targeted therapy.

In its broadest sense, a biological sample can be any sample that contains CTCs. A sample can comprise a bodily fluid such as blood; the soluble fraction of a cell preparation, or an aliquot of media in which cells were grown; a chromosome, an organelle, or membrane isolated or extracted from a cell; genomic DNA, RNA, or cDNA in solution or bound to a substrate; a cell; a tissue; a tissue print; a fingerprint; cells; skin, and the like. A biological sample obtained from a subject can be any sample that contains cells and encompasses any material in which CTCs can be detected. A sample can be, for example, whole blood, plasma, saliva or other bodily fluid or tissue that contains cells.

In particular embodiments, the biological sample is a blood sample. As described herein, a sample can be whole blood, more preferably peripheral blood or a peripheral blood cell fraction. As will be appreciated by those skilled in the art, a blood sample can include any fraction or component of blood, without limitation, T-cells, monocytes, neutrophiles, erythrocytes, platelets and microvesicles such as exosomes and exosome-like vesicles. In the context of this disclosure, blood cells included in a blood sample encompass any nucleated cells and are not limited to components of whole blood. As such, blood cells include, for example, both white blood cells (WBCs) as well as rare cells, including CTCs.

The samples of this disclosure can each contain a plurality of cell populations and cell subpopulation that are distinguishable by methods well known in the art (*e.g.,* FACS, immunohistochemistry). For example, a blood sample can contain populations of non-nucleated cells, such as erythrocytes (*e.g.,* 4-5 million/µl) or platelets (150,000-400,000 cells/µl), and populations of nucleated cells such as WBCs (*e.g.,* 4,500 - 10,000 cells/µl), CECs or CTCs (circulating tumor cells; *e.g.,* 2-800 cells/). WBCs may contain cellular subpopulations of, *e.g.,* neutrophils (2,500-8,000 cells/µl), lymphocytes (1,000-4,000 cells/µl), monocytes (100-700 cells/µl), eosinophils (50-500 cells/µl), basophils (25 - 100 cells/ µl) and the like. The samples of this disclosure are non-enriched samples, *i.e.,* they are not enriched for any specific population or subpopulation of nucleated cells. For example, non-enriched blood samples are not enriched for CTCs, WBC, B-cells, T-cells, NK-cells, monocytes, or the like.

In some embodiments the sample is a blood sample obtained from a healthy subject or a subject deemed to be at high risk for protate cancer or metastasis of existing prostate cancer based on art known clinically established criteria including, for example, age, race, family snd history. In some embodiments the blood sample is from a subject who has been diagnosed with prostate cancer and/or mCRPC based on tissue or liquid biopsy and/or surgery or clinical grounds. In some embodiments, the blood sample is obtained from a subject showing a clinical manifestation of prostate cancer and/or mCRPC well known in the art or who presents with any of the known risk factors for prostate cancer and/or mCRPC.

As used herein in the context of generating CTC data, the term "direct analysis" means that the CTCs are detected in the context of all surrounding nucleated cells present in the sample as opposed to after enrichment of the sample for CTCs prior to detection. In some embodiments, the methods comprise microscopy providing a field of view that includes both CTCs and at least 200 surrounding white blood cells (WBCs).

A fundamental aspect of the present disclosure is the unparalleled robustness of the disclosed methods with regard to the detection of CTCs. The rare event detection disclosed herein with regard to CTCs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis, but that instead compare enriched populations with inherently distorted contextual comparisons of rare events. The robustness of the direct analysis methods disclosed herein enables characterization of CTC, including subtypes of CTCs described herein, that allows for identification of phenotypes and heterogeneity that cannot be achievied with other CTC detection methods and that enables the analysis of biomarkers in the context of the claimed methods.

The methods for predicting *de novo* resistance to androgen receptor (AR) targeted therapy in a tumor of a prostate cancer patient can further encompass individual patient risk factors and imaging data, which includes any form of imaging modality known and used in the art, for example and without limitation, by X-ray computed tomography (CT), ultrasound, positron emission tomography (PET), electrical impedance tomography and magnetic resonance (MRI). It is understood that one skilled in the art can select an imaging modality based on a variety of art known criteria. As described herein, the methods can encompass one or more pieces of imaging data. In the methods disclosed herein, one or more individual risk factors can be selected from the group consisting of age, race, family history. It is understood that one skilled in the art can select additional individual risk factors based on a variety of art known criteria. As described herein, the methods can encompass one or more individual risk factors. Accordingly, biomarkers can include imaging data, individual risk factors and CTC data. As described herein, biomarkers also can include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (*e.g.,* glycoproteins, ribonucleoproteins, lipoproteins) as well as portions or fragments of a biological molecule.

CTC data can include both morphological features and immunofluorescent features. As will be understood by those skilled in the art, biomarkers can include a biological molecule, or a fragment of a biological molecule, the change and/or the detection of which can be correlated, individually or combined with other measurable features, with prostate cancer and/or mCRPC. CTCs, which can be present a single cells or in clusters of CTCs, are often epithelial cells shed from solid tumors and are present in very low concentrations in the circulation of subjects. Accordingly, detection of CTCs in a blood sample can be referred to as rare event detection. CTCs have an abundance of less than 1:1,000 in a blood cell population, *e.g.,* an abundance of less than 1:5,000, 1:10,000, 1:30,000, 1:50:000, 1:100,000, 1:300,000, 1:500,000, or 1:1,000,000. In some embodiments, the a CTC has an abundance of 1:50:000 to 1:100,000 in the cell population.

The samples of this disclosure may be obtained by any means, including, *e.g.,* by means of solid tissue biopsy or fluid biopsy (*see, e.g.,* Marrinucci D. et al., 2012, Phys. Biol. 9 016003). Briefly, in particular embodiments, the process can encompass lysis and removal of the red blood cells in a 7.5 mL blood sample, deposition of the remaining nucleated cells on specialized microscope slides, each of which accommodates the equivalent of roughly 0.5 mL of whole blood. A blood sample may be extracted from any source known to include blood cells or components thereof, such as venous, arterial, peripheral, tissue, cord, and the like. The samples may be processed using well known and routine clinical methods (*e.g.,* procedures for drawing and processing whole blood). In some embodiments, a blood sample is drawn into anti-coagulent blood collection tubes (BCT), which may contain EDTA or Streck Cell-Free DNA™. In other embodiments, a blood sample is drawn into CellSave® tubes (Veridex). A blood sample may further be stored for up to 12 hours, 24 hours, 36 hours, 48 hours, or 60 hours before further processing.

In some embodiments, the methods of this disclosure comprise an intitial step of obtaining a white blood cell (WBC) count for the blood sample. In certain embodiments, the WBC count may be obtained by using a HemoCue® WBC device (Hemocue, Ängelholm, Sweden). In some embodiments, the WBC count is used to determine the amount of blood required to plate a consistent loading volume of nucleated cells per slide and to calculate back the equivalent of CTCs per blood volume.

In some embodiments, the methods of this disclosure comprise an initial step of lysing erythrocytes in the blood sample. In some embodiments, the erythrocytes are lysed, *e.g.,* by adding an ammonium chloride solution to the blood sample. In certain embodiments, a blood sample is subjected to centrifugation following erythrocyte lysis and nucleated cells are resuspended, *e.g.,* in a PBS solution.

In some embodiments, nucleated cells from a sample, such as a blood sample, are deposited as a monolayer on a planar support. The planar support may be of any material, *e.g.,* any fluorescently clear material, any material conducive to cell attachment, any material conducive to the easy removal of cell debris, any material having a thickness of < 100 µm. In some embodiments, the material is a film. In some embodiments the material is a glass slide. In certain embodiments, the method encompasses an initial step of depositing nucleated cells from the blood sample as a monolayer on a glass slide. The glass slide can be coated to allow maximal retention of live cells (*See, e.g.,* Marrinucci D. et al., 2012, Phys. Biol. 9 016003). In some embodiments, about 0.5 million, 1 million, 1.5 million, 2 million, 2.5 million, 3 million, 3.5 million, 4 million, 4.5 million, or 5 million nucleated cells are deposited onto the glass slide. In some embodiments, the methods of this disclosure comprise depositing about 3 million cells onto a glass slide. In additional embodiments, the methods of this disclosure comprise depositing between about 2 million and about 3 million cells onto the glass slide. In some embodiments, the glass slide and immobilized cellular samples are available for further processing or experimentation after the methods of this disclosure have been completed.

In some embodiments, the methods of this disclosure comprise an initial step of identifying nucleated cells in the non-enriched blood sample. In some embodiments, the nucleated cells are identified with a fluorescent stain. In certain embodiments, the fluorescent stain comprises a nucleic acid specific stain. In certain embodiments, the fluorescent stain is diamidino-2-phenylindole (DAPI). In some embodiments, immunofluorescent staining of nucleated cells comprises pan cytokeratin (CK), cluster of differentiation (CD) 45 and DAPI. In some embodiments further described herein, CTCs comprise distinct immunofluorescent staining from surrounding nucleated cells. In some embodiments, the distinct immunofluorescent staining of CTCs comprises DAPI (+), CK (+) and CD 45 (-). In some embbodiments, the identification of CTCs further comprises comparing the intensity of pan cytokeratin fluorescent staining to surrounding nucleated cells. In some embodiments, the CTC data is generated by fluorescent scanning microscopy to detect immunofluorescent staining of nucleated cells in a blood sample. Marrinucci D. et al., 2012, Phys. Biol. 9 016003).

In particular embodiments, all nucleated cells are retained and immunofluorescently stained with monoclonal antibodies targeting cytokeratin (CK), an intermediate filament found exclusively in epithelial cells, a pan leukocyte specific antibody targeting the common leukocyte antigen CD45, and a nuclear stain, DAPI. The nucleated blood cells can be imaged in multiple fluorescent channels to produce high quality and high resolution digital images that retain fine cytologic details of nuclear contour and cytoplasmic distribution. While the surrounding WBCs can be identified with the pan leukocyte specific antibody targeting CD45, CTCs can be identified as DAPI (+), CK (+) and CD 45 (-). In the methods described herein, the CTCs comprise distinct immunofluorescent staining from surrounding nucleated cells.

In further embodiments, the CTC data includes traditional CTCs also known as high definition CTCs (HD-CTCs) . Traditional CTCs are CK positive, CD45 negative, contain an intact DAPI positive nucleus without identifiable apoptotic changes or a disrupted appearance, and are morphologically distinct from surrounding white blood cells (WBCs). DAPI (+), CK (+) and CD45 (-) intensities can be categorized as measurable features during HD-CTC enumeration as previously described (Figure 1). Nieva et al., Phys Biol 9:016004 (2012). The enrichment-free, direct analysis employed by the methods disclosed herein results in high sensitivity and high specificity, while adding high definition cytomorphology to enable detailed morphologic characterization of a CTC population known to be heterogeneous.

While CTCs can be identified as comprises DAPI (+), CK (+) and CD 45 (-) cells, the methods of the invention can be practiced with any other biomarkers that one of skill in the art selects for generating CTC data and/or identifying CTCs and CTC clusters. One skilled in the art knows how to select a morphological feature, biological molecule, or a fragment of a biological molecule, the change and/or the detection of which can be correlated with a CTC. Molecule biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins). The term also encompasses portions or fragments of a biological molecule, for example, peptide fragment of a protein or polypeptide

A person skilled in the art will appreciate that a number of methods can be used to generate CTC data, including microscopy based approaches, including fluorescence scanning microscopy (*see, e.g.,* Marrinucci D. et al., 2012, Phys. Biol. 9 016003), mass spectrometry approaches, such as MS/MS, LC-MS/MS, multiple reaction monitoring (MRM) or SRM and product-ion monitoring (PIM) and also including antibody based methods such as immunofluorescence, immunohistochemistry, immunoassays such as Western blots, enzyme-linked immunosorbant assay (ELISA), immunopercipitation, radioimmunoassay, dot blotting, and FACS. Immunoassay techniques and protocols are generally known to those skilled in the art (Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and non-competitive immunoassays, can be used (Self et al., Curr. Opin. Biotechnol., 7:60-65 (1996), *see also* John R. Crowther, The ELISA Guidebook, 1st ed., Humana Press 2000, ISBN 0896037282 and, An Introduction to Radioimmunoassay and Related Techniques, by Chard T, ed., Elsevier Science 1995, ISBN 0444821198).

A person of skill in the art will futher appreciate that the presence or absence of biomarkers may be detected using any class of marker-specific binding reagents known in the art, including, *e.g.,* antibodies, aptamers, fusion proteins, such as fusion proteins including protein receptor or protein ligand components, or biomarker-specific small molecule binders. In some embodiments, the presence or absence of CK or CD45 is determined by an antibody.

The antibodies of this disclosure bind specifically to a biomarker. The antibody can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). The antibody can be any immunoglobulin or derivative thereof, whether natural or wholly or partially synthetically produced. All derivatives thereof which maintain specific binding ability are also included in the term. The antibody has a binding domain that is homologous or largely homologous to an immunoglobulin binding domain and can be derived from natural sources, or partly or wholly synthetically produced. The antibody can be a monoclonal or polyclonal antibody. In some aspects, an antibody is a single chain antibody. Those of ordinary skill in the art will appreciate that antibody can be provided in any of a variety of forms including, for example, humanized, partially humanized, chimeric, chimeric humanized, *etc.* The antibody can be an antibody fragment including, but not limited to, Fab, Fab', F(ab')2, scFv, Fv, dsFv diabody, and Fd fragments. The antibody can be produced by any means. For example, the antibody can be enzymatically or chemically produced by fragmentation of an intact antibody and/or it can be recombinantly produced from a gene encoding the partial antibody sequence. The antibody can comprise a single chain antibody fragment. Alternatively or additionally, the antibody can comprise multiple chains which are linked together, for example, by disulfide linkages, and any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

A detectable label can be used in the methods described herein for direct or indirect detection of the biomarkers when generating CTC data in the methods of the invention. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers in the methods of the invention. Suitable detectable labels include, but are not limited to, fluorescent dyes (*e.g.,* fluorescein, fluorescein isothiocyanate (FITC), Oregon Green™, rhodamine, Texas red, tetrarhodimine isothiocynate (TRITC), Cy3, Cy5, Alexa Fluor® 647, Alexa Fluor® 555, Alexa Fluor® 488), fluorescent markers (*e.g.,* green fluorescent protein (GFP), phycoerythrin, etc.), enzymes (*e.g.,* luciferase, horseradish peroxidase, alkaline phosphatase, etc.), nanoparticles, biotin, digoxigenin, metals, and the like.

For mass-sectrometry based analysis, differential tagging with isotopic reagents, *e.g.,* isotope-coded affinity tags (ICAT) or the more recent variation that uses isobaric tagging reagents, iTRAQ (Applied Biosystems, Foster City, Calif.), followed by multidimensional liquid chromatography (LC) and tandem mass spectrometry (MS/MS) analysis can provide a further methodology in practicing the methods of this disclosure.

A chemiluminescence assay using a chemiluminescent antibody can be used for sensitive, non-radioactive detection of proteins. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase, beta.-galactosidase are well known in the art.

A signal from the direct or indirect label can be analyzed, for example, using a microscope, such as a fluorescence microscope or a fluorescence scanning microscope. Alternatively, a spectrophotometer can be used to detect color from a chromogenic substrate; a radiation counter to detect radiation such as a gamma counter for detection of ¹²⁵I; or a fluorometer to detect fluorescence in the presence of light of a certain wavelength. If desired, assays used to practice the methods of this disclosure can be automated or performed robotically, and the signal from multiple samples can be detected simultaneously.

In some embodiments, the biomarkers are immunofluorescent markers. In some embodiments, the immunofluorescent makers comprise a marker specific for epithelial cells In some embodiments, the immunofluorescent makers comprise a marker specific for white blood cells (WBCs). In some embodiments, one or more of the immunofluorescent markers comprise CD 45 and CK.

In some embodiments, the presence or absence of immunofluorescent markers in nucleated cells, such as CTCs or WBCs, results in distinct immunofluorescent staining patterns. Immunofluorescent staining patterns for CTCs and WBCs may differ based on which epithelial or WBC markers are detected in the respective cells. In some embodiments, determining presence or absence of one or more immunofluorescent markers comprises comparing the distinct immunofluorescent staining of CTCs with the distinct immunofluorescent staining of WBCs using, for example, immunofluorescent staining of CD45, which distinctly identifies WBCs. There are other detectable markers or combinations of detectable markers that bind to the various subpopulations of WBCs. These may be used in various combinations, including in combination with or as an alternative to immunofluorescent staining of CD45.

In some embodiments, CTCs comprise distinct morphological characteristics compared to surrounding nucleated cells. In some embodiments, the morphological characteristics comprise nucleus size, nucleus shape, cell size, cell shape, and/or nuclear to cytoplasmic ratio. In some embodiments, the method further comprises analyzing the nucleated cells by nuclear detail, nuclear contour, presence or absence of nucleoli, quality of cytoplasm, quantity of cytoplasm, intensity of immunofluorescent staining patterns. A person of ordinary skill in the art understands that the morphological characteristics of this disclosure may include any feature, property, characteristic, or aspect of a cell that can be determined and correlated with the detection of a CTC.

CTC data can be generated with any microscopic method known in the art. In some embodiments, the method is performed by fluorescent scanning microscopy. In certain embodiments the microscopic method provides high-resolution images of CTCs and their surrounding WBCs (*see, e.g.,* Marrinucci D. *et al.,* 2012, Phys. Biol. 9 016003)). In some embodiments, a slide coated with a monolayer of nucleated cells from a sample, such as a non-enriched blood sample, is scanned by a fluorescent scanning microscope and the fluorescence intensities from immunofluorescent markers and nuclear stains are recorded to allow for the determination of the presence or absence of each immunofluorescent marker and the assessment of the morphology of the nucleated cells. In some embodiments, microscopic data collection and analysis is conducted in an automated manner.

In some embodiments, a CTC data includes detecting one or more biomarkers, for example, CK and CD 45. A biomarker is considered "present" in a cell if it is detectable above the background noise of the respective detection method used (*e.g.,* 2-fold, 3-fold, 5-fold, or 10-fold higher than the background; *e.g.,* 2σ or 3σ over background). In some embodiments, a biomarker is considered "absent" if it is not detectable above the background noise of the detection method used (*e.g.,* <1.5-fold or <2.0-fold higher than the background signal; *e.g.,* <1.5σ or <2.0σ over background).

In some embodiments, the presence or absence of immunofluorescent markers in nucleated cells is determined by selecting the exposure times during the fluorescence scanning process such that all immunofluorescent markers achieve a pre-set level of fluorescence on the WBCs in the field of view. Under these conditions, CTC-specific immunofluorescent markers, even though absent on WBCs are visible in the WBCs as background signals with fixed heights. Moreover, WBC-specific immunofluorescent markers that are absent on CTCs are visible in the CTCs as background signals with fixed heights. A cell is considered positive for an immunofluorescent marker (*i.e.,* the marker is considered present) if its fluorescent signal for the respective marker is significantly higher than the fixed background signal (*e.g.,* 2-fold, 3-fold, 5-fold, or 10-fold higher than the background; *e.g.,* 2σ or 3σ over background). For example, a nucleated cell is considered CD 45 positive (CD 45⁺) if its fluorescent signal for CD 45 is significantly higher than the background signal. A cell is considered negative for an immunofluorescent marker (*i.e.,* the marker is considered absent) if the cell's fluorescence signal for the respective marker is not significantly above the background signal (*e.g.,* <1.5-fold or <2.0-fold higher than the background signal; *e.g.,* <1.5σ or <2.0σ over background).

Typically, each microscopic field contains both CTCs and WBCs. In certain embodiments, the microscopic field shows at least 1, 5, 10, 20, 50, or 100 CTCs. In certain embodiments, the microscopic field shows at least 10, 25, 50, 100, 250, 500, or 1,000 fold more WBCs than CTCs. In certain embodiments, the microscopic field comprises one or more CTCs or CTC clusters surrounded by at least 10, 50, 100, 150, 200, 250, 500, 1,000 or more WBCs.

In some embodiments of the methods described herein, generation of the CTC data comprises enumeration of CTCs that are present in the blood sample. In some embodiments, the methods described herein encompass detection of at least 1.0 CTC/mL of blood, 1.5 CTCs/mL of blood, 2.0 CTCs/mL of blood, 2.5 CTCs/mL of blood, 3.0 CTCs/mL of blood, 3.5 CTCs/mL of blood, 4.0 CTCs/mL of blood, 4.5 CTCs/mL of blood, 5.0 CTCs/mL of blood, 5.5 CTCs/mL of blood, 6.0 CTCs/mL of blood, 6.5 CTCs/mL of blood, 7.0 CTCs/mL of blood, 7.5 CTCs/mL of blood, 8.0 CTCs/mL of blood, 8.5 CTCs/mL of blood, 9.0 CTCs/mL of blood, 9.5 CTCs/mL of blood, 10 CTCs/mL of blood, or more.

In some embodiments of methods described herein, generation of the CTC data comprises detecting distinct subtypes of CTCs, including non-traditional CTCs. In some embodiments, the methods described herein encompass detection of at least 0.1 CTC cluster/mL of blood, 0.2 CTC clusters/mL of blood, 0.3 CTC clusters/mL of blood, 0.4 CTC clusters/mL of blood, 0.5 CTC clusters/mL of blood, 0.6 CTC clusters/mL of blood, 0.7 CTC clusters/mL of blood, 0.8 CTC clusters/mL of blood, 0.9 CTC clusters/mL of blood, 1 CTC cluster/mL of blood, 2 CTC clusters/mL of blood, 3 CTC clusters/mL of blood, 4 CTC clusters/mL of blood, 5 CTC clusters/mL of blood, 6 CTC clusters/mL of blood, 7 CTC clusters/mL of blood, 8 CTC clusters/mL of blood, 9 CTC clusters/mL of blood, 10 clusters/mL or more. In a particular embodiment, the methods described herein encompass detection of at least 1 CTC cluster/mL of blood.

In some embodiments, the disclosed methods for prospectively identifying *de novo* resistance to AR targeted therapies in a mCRPC patient encompass the use of a predictive model. In further embodiments, the disclosed methods for prospectively identifying *de novo* resistance to AR targeted therapies in a mCRPC patient encompass comparing a measurable feature with a reference feature. As those skilled in the art can appreciate, such comparison can be a direct comparison to the reference feature or an indirect comparison where the reference feature has been incorporated into the predictive model. In further embodiments, analyzing a measurable feature to prospectively identify *de novo* resistance to AR targeted therapies in a mCRPC patient encompasses one or more of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, or a combination thereof. In particular embodiments, the analysis comprises logistic regression. In additional embodiments, the identification of *de novo* resistance to AR targeted therapies in a mCRPC patient is expressed as a risk score.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms and other methods known to those skilled in the art.

Classification can be made according to predictive modeling methods that set a threshold for determining the probability that a sample belongs to a given class. The probability preferably is at least 50%, or at least 60%, or at least 70%, or at least 80%, or at least 90% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, *e.g.* AUROC (area under the ROC curve) or accuracy, of a particular value, or range of values. Area under the curve measures are useful for comparing the accuracy of a classifier across the complete data range. Classifiers with a greater AUC have a greater capacity to classify unknowns correctly between two groups of interest. ROC analysis can be used to select the optimal threshold under a variety of clinical circumstances, balancing the inherent tradeoffs that exist between specificity and sensitivity. In some embodiments, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher.

As is known in the art, the relative sensitivity and specificity of a predictive model can be adjusted to favor either the specificity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about 0.7, at least about 0.75, at least about 0.8, at least about 0.85, at least about 0.9, or higher.

The raw data can be initially analyzed by measuring the values for each measurable feature or biomarker, usually in triplicate or in multiple triplicates. The data can be manipulated, for example, raw data can be transformed using standard curves, and the average of triplicate measurements used to calculate the average and standard deviation for each patient. These values can be transformed before being used in the models, *e.g.* log-transformed, Box-Cox transformed (Box and Cox, Royal Stat. Soc., Series B, 26:211-246(1964). The data are then input into a predictive model, which will classify the sample according to the state. The resulting information can be communicated to a patient or health care provider.

In some embodiments, the method disclosed herein for prospectively identifying *de novo* resistance to AR targeted therapies in a mCRPC patient has a specificity of >60%, >70%, >80%, >90% or higher. In additional embodiments, the method prospectively identifying *de novo* resistance to AR targeted therapies in a mCRPC patient has a specificity >90% at a classification threshold of 7.5 CTCs/mL of blood.

As will be understood by those skilled in the art, an analytic classification process can use any one of a variety of statistical analytic methods to manipulate the quantitative data and provide for classification of the sample. Examples of useful methods include, without limitation, linear discriminant analysis, recursive feature elimination, a prediction analysis of microarray, a logistic regression, a CART algorithm, a FlexTree algorithm, a LART algorithm, a random forest algorithm, a MART algorithm, and machine learning algorithms.

From the foregoing description, it will be apparent that variations and modifications can be made to the invention described herein to adopt it to various usages and conditions.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

The following examples are provided by way of illustration, not limitation.

### EXAMPLES

### Example 1. Characteristics of De Novo Resistance to Androgen Targeting Therapeutics (AR Tx) through Circulating Tumor Cells (CTCs) analyses in metastatic Castration Resistant Prostate Cancer (mCRPC) patients

This example demonstrates that patients not responding to AR targeted therapies demonstrated greater heterogeneity of CTC subpopulations compared to patients who responded to AR targeted therapies.

Sample evaluation for CTCs was performed as reported previously using the Epic Sciences Platform. Marrinucci et al. Phys Biol 9:016003, 2012. Figure 2 shows a schematic of Epic's CTC collection and detection process, which flows as follows: (1) Blood lysed, nucleated cells from blood sample placed onto slides; (2) Slides stored in -80C biorepository; (3) Slides stained with CK, CD45, DAPI and AR; (4) Slides scanned; (5) Multi-parametric digital pathology algorithms run, and (6) Software and human reader confirmation of CTCs & quantitation of biomarker expression.. 85 blood samples were collected from mCRPC patients immediately prior to treatment with Abiraterone Acetate + Prednisone (A), Enzalutamide (E) or taxane (T) and total CTCs, specific CTC subpopulations and CTC AR protein expression were examined. Results were correlated with response patterns to AR signaling directed therapies measured by PSA changes (Figure 1). Patients on A, E or T were classified according to Figure 1 as: Responders (n=39) which included: true response (n=15) acquired resistance (n=24). Non responders (n=46): *de novo* resistance

Figures 1 through 7 and 15 describe further experimental details.

Blood samples underwent hemolysis, centrifugation, re-suspension and plating onto slides, followed by -80°C storage. Prior to analysis, slides were thawed, labeled by immunofluorescence (pan cytokeratin, CD45, DAPI and AR) and imaged by automated fluoroscopy then manual validation by a pathologist-trained technician (MSL). Marrinucci et al. Phys Biol 9:016003, 2012. DAPI (+), CK (+) and CD45 (-) intensities were categorized as features during HD-CTC enumeration as previously described.

The study described in this example demonstrates that there is no significant difference in the number of CTCs/ 7.5 mL or non-traditional CTCs/ 7.5 mL of patient blood between the AR Therapy or Taxane outcome. Thus, CTC/7.5 mL or non-traditional CTC/7.5 mL frequency does not predict Abiraterone, Enzalutamideor Taxane resistance

The study also shows that sensitivity to Abiraterone or Enzalutamide is assessable from baseline blood draw through the single cell CTC measurement of:
1. CTC Heterogeneity
2. Frequency of cytokeratin positive, AR N-terminal positive CTCs with prominent nucleoli morphology
3. AR C-terminal loss

The study described in this example demonstrates that CTC profile of sensitivity to Abiraterone and Enzalutamide resistant disease does not predict sensitivity to Taxane therapy, confirming that the methods disclosed enable treatment selection based on the predictive signatures described herein.

The study also shows that patients not responding to AR targeted therapies demonstrated greater heterogeneity of CTC subpopulations compared to patients who responded to AR targeted therapies.

The study further demonstrates that single CTC characterization to measure heterogeneity of CTC subpopulations can be utilized to predict resistance to AR targeted therapies.

The study also demonstrates that epithelial expression, Androgen N-terminal staining and morphologic assessment of CTCs can be utilized to predict response to AR targeted therapies.

The study also shows that, while the signature for AR therapy resistance is not associated with resistance to Taxane, optimization of a signature for AR therapy resistance and Taxane response can be achieved.

### Example 2. Characterization of Circulating Tumor Cells (CTCs) in metastatic Castration Resistant Prostate Cancer (mCRPC) patients in First, Second & Third Line Systemic Therapies

New approved androgen signaling directed therapies (AR Therapy), including Abiraterone Acetate plus Prednisone (A) and Enzalutamide (E), prolong survival in patients (pts) with mCRPC. This example demonstrates that, used sequentially, response to E given after A is of shorter duration and less frequent. Targeting patients more likely to benefit from novel specific therapies is necessary to develop more effective treatment(s). This example further shows the evolution of CTC phenotypes along with AR expression and AR localization in pts receiving the 1^{st}, 2^{nd}, and 3^{rd} lines of systemic therapy for mCRPC.

Sample evaluation for CTCs was performed as reported previously using the Epic Sciences Platform. Marrinucci et al. Phys Biol 9:016003, 2012. Figure 2 shows a schematic of Epic's CTC collection and detection process, which flows as follows: (1) Blood lysed, nucleated cells from blood sample placed onto slides; (2) Slides stored in -80C biorepository; (3) Slides stained with CK, CD45, DAPI and AR; (4) Slides scanned; (5) Multi-parametric digital pathology algorithms run, and (6) Software and human reader confirmation of CTCs & quantitation of biomarker expression.. 117 samples from 112 unique patients (58 with no prior therapy, 33 who failed 1 prior therapy and 27 who failed 2+ prior therapy) had blood collection for CTC analysis utilizing the Epic Sciences platform. Epic analysis included identification CTC subtypes as described below: In addition, CTC AR expression, AR subcellular localization and observed cell morphology (size, nucleoli, CK speckles) were evaluated. CTC and patient data from all patient samples were compared across treatment groups (therapy naive, failed 1^{st} line therapy or failed 1^{st} and 2^{nd} line therapy) and subgroups representative of specific clinical regiments.

Figures 8 through 14 and 16 describe further experimental details.

The study described in this example demonstrates that overall number of traditional and non-traditional CTCs increases as the number of systemic therapies administered increases. (Therapy naive, failed 1^{st} line, or failed 1^{st} and 2nd line).

The results further demonstrate that the number of CTCs, CTC subtypes and CTC phenotypes is highly variable at each of the treatment decision points studied. (Therapy naive, failed 1^{st} line, or failed 1^{st} and 2^{nd} line).

The results also show that heterogeneity of non-traditional CTC subpopulations increases with 1^{st}, 2^{nd} or 3^{rd} lines of systemic therapy (Therapy naive, failed 1^{st} line, or failed 1^{st} and 2^{nd} line).

The results also demonstrate that more unique CTC phenotypes are observed as successive therapies are given: (a) Increase CTC phenotypes include: Nucleoli CTCs, CTC Clusters, CK- CTCs, CK Speckled CTCs, Small CTCs, AR Nuclear CTCs, AR Cytoplasmic CTCs. The increase in phenotypes is consistent with a more heterogeneous tumor. The results also demonstrate that t racking the evolution of and direct characterization of the phenotypes enables methods disclosed herein for more effective treatment management.

The results further demonstrate that the percentage of patients with CTC phenotypes associated with *de novo* resistance increases as the number of systemic therapies administered increases.

The results also show an increasing prevalence of AR Nuclear CTCs after A or E may identify patients with constitutively active androgen signaling.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

## Claims

1. A method of predicting *de novo* resistance to androgen receptor (AR) targeted therapy in a tumor of a prostate cancer patient comprising
(a) performing a direct analysis comprising immunofluorescent staining and morphological characteristization of nucleated cells in a blood sample obtained from the patient to generate circulating tumor cell (CTC) data, wherein the analysis comprises determining a measurable feature of a panel of traditional and non-traditional CTC biomarkers for *de novo* resistance to androgen receptor (AR) targeted therapy,
wherein the biomarkers comprise (1) CTC heterogeneity, (2) frequency of cytokeratin positive (CK+), AR N-terminal positive CTCs with prominent nucleoli morphology, and (3) frequency of AR C-terminal truncated CTCs, and
wherein the CTC heterogeneity comprises biomarkers selected from the group consisting of traditional CTCs, CTC clusters, CK- CTCs, small CTCs, nucleoli+ CTCs, CK speckled CTCs and
(b) evaluating the CTC data to determine the probability of *de novo* resistance to the AR targeted therapy in the tumor of the prostate cancer patient,
wherein (1) greater CTC heterogeneity, (2) increased frequency of CK+, AR N-terminal positive CTCs with prominent nucleoli morphology, or (3) increased frequency of AR C-terminal truncated CTCs, compared to patients responsive to AR therapy, predicts *de novo* resistance to AR therapy

2. The method of claim 1, wherein the immunofluorescent staining comprises staining of nucleated cells comprising pan cytokeratin, cluster of differentiation (CD) 45, diamidino-2-phenylindole (DAPI) and AR.

3. The method of any one of claim 1 or 2 comprising an initial step of depositing the nucleated cells as a monolayer onto a slide.

4. The method of any one of claims 1-3, wherein the prostate cancer is metastatic castration resistant prostate cancer (mCRPC).

5. The method of any one of claims 1-4, wherein the CTC data is generated by fluorescent scanning microscopy.

6. The method of claim 5, wherein the microscopy provides a field of view comprising both CTCs and at least 200 surrounding white blood cells (WBCs).

7. The method of any one of claims 1-6, wherein the CTC data is generated by assessing at least 4 million of the nucleated cells.

8. The method of claim 5, wherein the CTCs comprise distinct immunofluorescent staining from surrounding nucleated cells; or wherein the CTCs comprise distinct morphological characteristics compared to surrounding nucleated cells.

9. The method of claim 8, wherein the morphological characteristics comprise one or more of the group consisting of nucleus size, nucleus shape, presence of holes in nucleus, cell size, cell shape and nuclear to cytoplasmic ratio, nuclear detail, nuclear contour, presence or absence of nucleoli, quality of cytoplasm and quantity of cytoplasm.

10. The method of any one of claims 1-9, wherein the identification of CTCs further comprises comparing intensity of pan cytokeratin fluorescent staining to surrounding nucleated cells.

11. The method of any one of claims 1-10, further comprising:
(a) an initial step of obtaining a white blood cell (WBC) count for the blood sample;
(b) an initial step of lysing erythrocytes in the blood sample; and/or
(c) an initial step of depositing nucleated cells from the blood sample as a monolayer on a glass slide.

12. The method of claim 11, further comprising depositing between about 2 million and about 3 million cells onto the glass slide.

13. The method of any one of claims 1-12, wherein the generation of the CTC data comprises enumeration of CTCs in the blood sample.

14. The method of any one of claims 1-13, wherein the measurable features are analyzed using a predictive model.

15. The method of claim 14, wherein the model is a multivariate model.

## Patentansprüche

1. Verfahren zur Prognose einer *de novo*-Resistenz gegenüber einer auf den Androgenrezeptor (AR) gerichteten Therapie in einem Tumor eines Prostatakrebspatienten, umfassend
(a) Durchführen einer direkten Analyse, umfassend die Immunfluoreszenzfärbung und die morphologische Charakterisierung von kernhaltigen Zellen in einer Blutprobe, die von dem Patienten erhalten wurde, um Daten über zirkulierende Tumorzellen (CTC) zu erzeugen, wobei die Analyse das Bestimmen eines messbaren Merkmals eines Satzes an traditionellen und nicht-traditionellen CTC-Biomarkern für eine *de* novo-Resistenz gegenüber einer gezielten Androgenrezeptor (AR)-Therapie umfasst,
wobei die Biomarker (1) CTC-Heterogenität, (2) Häufigkeit von Cytokeratinpositiven (CK+), AR N-terminus positiven CTC mit prominenter Nukleoli-Morphologie und (3) Häufigkeit von AR C-terminus trunkierten CTC umfassen, und
wobei die CTC-Heterogenität Biomarker umfasst, die ausgewählt sind aus der Gruppe bestehend aus traditionellen CTC, CTC-Clustern, CK- CTC, kleinen CTC, Nucleoli+ CTC, CK gesprenkelte CTC; und
(b) Auswerten der CTC-Daten, um die Wahrscheinlichkeit einer *de novo*-Resistenz gegenüber der gezielten AR-Therapie im Tumor des Prostatakrebspatienten zu bestimmen,
wobei (1) eine größere CTC-Heterogenität, (2) eine erhöhte Häufigkeit von CK+, AR N-terminus positiven CTCs mit prominenter Nukleoli-Morphologie oder (3) eine erhöhte Häufigkeit von AR C-terminus trunkierten CTC im Vergleich zu Patienten, die auf die AR-Therapie ansprechen, eine *de novo* Resistenz gegenüber der AR-Therapie vorhersagt.

2. Verfahren nach Anspruch 1, wobei die Immunfluoreszenzfärbung die Färbung von kernhaltigen Zellen umfasst, die Pan-Cytokeratin, Cluster of Differentiation (CD) 45, Diamidino-2-Phenylindol (DAPI) und AR umfassen.

3. Verfahren nach einem der Ansprüche 1 oder 2, umfassend einen ersten Schritt des Ablagerns der kernhaltigen Zellen als Monoschicht auf einem Objektträger.

4. Verfahren nach einem der Ansprüche 1-3, wobei der Prostatakrebs ein metastasierter, kastrationsresistenter Prostatakrebs (mCRPC) ist.

5. Verfahren nach einem der Ansprüche 1-4, wobei die CTC-Daten durch Fluoreszenz-Rastermikroskopie erzeugt werden.

6. Verfahren nach Anspruch 5, wobei die Mikroskopie ein Sichtfeld liefert, das sowohl CTCs als auch mindestens 200 umgebende weiße Blutkörperchen (WBK) umfasst.

7. Verfahren nach einem der Ansprüche 1-6, wobei die CTC-Daten durch Bewertung von mindestens 4 Millionen der kernhaltigen Zellen erzeugt werden.

8. Verfahren nach Anspruch 5, wobei die CTC eine unterschiedliche immunfluoreszierende Färbung zu den umgebenden kernhaltigen Zellen umfassen; oder wobei die CTC im Vergleich zu den umgebenden kernhaltigen Zellen unterschiedliche morphologische Merkmale umfassen.

9. Verfahren nach Anspruch 8, wobei die morphologischen Merkmale eins oder mehrere der Gruppe bestehend aus Kerngröße, Kernform, Vorhandensein von Löchern im Kern, Zellgröße, Zellform und Kern-zu-Zytoplasma-Verhältnis, Kerndetail, Kernkontur, Vorhandensein oder Fehlen von Nukleoli, Qualität des Zytoplasmas und Menge des Zytoplasmas umfassen.

10. Verfahren nach einem der Ansprüche 1-9, wobei die Identifizierung von CTC ferner den Vergleich der Intensität der Pan-Cytokeratin-Fluoreszenzfärbung mit umgebenden kernhaltigen Zellen umfasst.

11. Verfahren für einen der Ansprüche 1-10, ferner umfassend:
(a) einen ersten Schritt zum Erzielen einer Anzahl weißer Blutkörperchen (WBK) für die Blutprobe;
(b) einen ersten Schritt der Lyse von Erythrozyten in der Blutprobe; und/oder
(c) einen ersten Schritt der Ablagerung von kernhaltigen Zellen aus der Blutprobe als Monoschicht auf einem Glasobjektträger.

12. Verfahren nach Anspruch 11 ferner umfassend die Ablagerung von etwa 2 Millionen bis etwa 3 Millionen Zellen auf dem Glasobjektträger.

13. Verfahren nach einem der Ansprüche 1-12, wobei die Erzeugung der CTC-Daten die Aufzählung der CTC in der Blutprobe umfasst.

14. Verfahren nach einem der Ansprüche 1-13, wobei die messbaren Merkmale unter Verwendung eines Prognosemodells analysiert werden.

15. Verfahren nach Anspruch 14, wobei das Modell ein multivariates Modell ist.

## Revendications

1. Méthode de prédiction de la résistance *de novo* à la thérapie ciblant les récepteurs androgènes (AR) dans une tumeur d'un patient atteint de cancer de la prostate, comprenant
(a) effectuer une analyse directe comprenant la coloration immunofluorescente et la caractérisation morphologique des cellules nucléées dans un échantillon de sang obtenu du patient pour générer des données sur les cellules tumorales circulantes (CTC), dans laquelle l'analyse comprend la détermination d'une caractéristique mesurable d'un panel de biomarqueurs CTC traditionnels et non traditionnels de la résistance *de novo* à la thérapie ciblant les récepteurs androgènes (AR),
dans laquelle les biomarqueurs comprennent (1) l'hétérogénéité des CTC, (2) la fréquence des CTC à cytokératine positive (CK+), AR N-terminal positif avec une morphologie de nucléole proéminente, et (3) la fréquence des CTC AR C-terminal tronqué, et
dans laquelle l'hétérogénéité des CTC comprend des biomarqueurs choisis parmi le groupe constitué par les CTC traditionnels, les groupes de CTC, les CTC CK-, les petits CTC, les CTC nucleoli+, les CTC tachetées avec CK; et
(b) évaluer les données de la CTC pour déterminer la probabilité d'une résistance *de novo* à la thérapie ciblant les AR dans la tumeur du patient atteint d'un cancer de la prostate,
dans laquelle (1) une plus grande hétérogénéité des CTC, (2) une fréquence accrue de CTC CK+, AR N-terminal positif avec une morphologie de nucléole importante, ou (3) une fréquence accrue de CTC AR C-terminal tronqué, par rapport aux patients répondant à la thérapie AR, prédit la résistance *de novo* à la thérapie AR

2. Méthode de la revendication 1, dans laquelle la coloration immunofluorescente comprend la coloration de cellules nucléées comprenant la pancytokératine, le cluster de différenciation (CD) 45, le diamidino-2-phénylindole (DAPI) et les AR

3. Méthode de l'une des revendications 1 ou 2 comprenant une étape initiale de dépôt des cellules nucléées en monocouche sur une lame.

4. Méthode de l'une des revendications 1 à 3, dans laquelle le cancer de la prostate est un cancer de la prostate métastatique résistant à la castration (CRPCm).

5. Méthode de l'une des revendications 1 à 4, dans laquelle les données CTC sont générées par microscopie à balayage fluorescent.

6. Méthode de la revendication 5, dans laquelle la microscopie fournit un champ de vision comprenant à la fois des CTC et au moins 200 globules blancs (GB) environnants.

7. Méthode de l'une des revendications 1 à 6, dans laquelle les données CTC sont générées par l'évaluation d'au moins 4 millions de cellules nucléées.

8. Méthode de la revendication 5, dans laquelle les CTC comprennent une coloration immunofluorescente distincte des cellules nucléées environnantes ; ou dans laquelle les CTC comprennent des caractéristiques morphologiques distinctes par rapport aux cellules nucléées environnantes.

9. Méthode de la revendication 8, dans laquelle les caractéristiques morphologiques comprennent un ou plusieurs du groupe constitué par la taille du noyau, la forme du noyau, la présence de trous dans le noyau, la taille de cellule, la forme de cellule et le rapport nucléo-cytoplasmique, le détail nucléaire, le contour du noyau, la présence ou l'absence de nucléoles, la qualité du cytoplasme et la quantité de cytoplasme.

10. Méthode de l'une quelconque des revendications 1 à 9, dans laquelle l'identification des CTC comprend en outre la comparaison de l'intensité de la coloration fluorescente de la pancytokératine aux cellules nucléées environnantes.

11. Méthode de l'une des revendications 1 à 10, comprenant en outre :
(a) une étape initiale d'obtenir un nombre de globules blancs (GB) pour l'échantillon de sang ;
b) une étape initiale de lyser les érythrocytes dans l'échantillon de sang ; et/ou
(c) une étape initiale de déposer les cellules nucléées de l'échantillon de sang en monocouche sur une lame de verre.

12. Méthode de la revendication 11, comprenant en outre le dépôt d'environ 2 à 3 millions de cellules sur la lame de verre.

13. Méthode de l'une des revendications 1 à 12, dans laquelle la génération des données CTC comprend le dénombrement des CTC dans l'échantillon de sang.

14. Méthode de l'une des revendications 1 à 13, dans laquelle les caractéristiques mesurables sont analysées à l'aide d'un modèle prédictif.

15. Méthode de la revendication 14, dans laquelle le modèle est un modèle multivarié.
